# EUROPEAN PATENT SPECIFICATION

(11) **EP 0 245 945 B1**
(45) Date of publication and mention of the grant of the patent: **10.11.1993**
(21) Application number: 87303040.7
(22) Date of filing: 08.04.1987
(51) Int. Cl.: C12P 19/34, C12N 15/00, C12M 1/00, B01L 11/00, G01N 35/00

(54) **Automated nucleic acid extractor**
Automatisches Gerät zum Extrahieren von Nukleinsäuren
Appareil automatique pour l'extraction des acides nucléiques

(30) Priority: 11.04.1986 US 850869
(43) Date of publication of application: 19.11.1987
(73) Proprietor: APPLIED BIOSYSTEMS, INC., Foster City California 94404 (US)
(72) Inventor: Cathcart, Guy Richard, Berkerley CA 94707 (US); Mayrand, P. Eric, Pacifica, CA 94044 (US); Nordman, Eric S., Palo Alto CA 94301 (US)
(74) Representative: West, Alan Harry

(56) References cited:
- EP-A- 0 215 533
- DE-A- 1 808 800
- GB-A- 2 018 927
- US-A- 3 389 133
- CHEMICAL ABSTRACTS, vol. 67, no. 6, 7th August 1967, abstract no. 28932n, N.M. KREDICH et al.: "An inexpensive Luer-fitting chromatographic column."
- METHODS OF ENZYMOLOGY, vol. 152, 1987, Academic Press Inc., San Diego, California, US, pp. 33-39, pp. 180-183

## Description

This invention relates to the isolation and purification of nucleic acids from cells, and particularly to apparatus for automatically achieving such isolation.

Reference is directed to EP 0215533 belonging to the same applicant which is also concerned with an automated nucleic acid extractor. The contents of this previous application are incorporated herein by reference.

One of the first steps in the in vitro manipulation of nucleic acids involves their isolation. For example, relatively pure samples of genomic DNA are required in order to perform tests for genetic disease and recombinant technology requires isolation of both the vector DNA and the DNA to be cloned.

As a general rule, DNA does not exist as a free molecule in a cell, but instead exists as a complex association of DNA, RNA and proteins. This is a consequence of the role of DNA as the carrier of the genetic information. The DNA is used as a template for the production of messenger RNA, which is translated by the ribosome into protein. Proteins directly involved in the process of gene expression, such as RNA polymerase and regulatory proteins, interact with DNA in vivo to form nucleo-protein complexes, DNA polymerase, DNA ligase, various unwinding and supercoiling enzymes, recombination and repair enzymes, and those proteins involved in the initiation or maintenance of DNA replication are also associated with DNA in vivo and hence complicate the isolation of pure DNA. Because of this complex association of DNA with these other proteins and nucleic acids, the purification (isolation) approach for obtaining DNA can generally be thought of as a three step process: (1) releasing soluble, high molecular weight DNA through the disrupted cell walls and membranes; (2) dissociating DNA-protein complexes by protein denaturation or proteolysis: and (3) separating DNA from the other macromolecules.

Within this process, DNA of bacterial origin (prokaryotic DNA) is typically purified by different methods, depending on whether the DNA is chromosomal DNA, the bacterial cell wall is generally weakened by freeze-thawing or by treatment with the enzyme lysozyme and the chelating agent ethylenediaminetetraacetic acid (EDTA). Cell lysis is accomplished by the addition of a detergent such as sodium dodecyl sulfate (SDS) in a buffered saline solution. Following lysis, the solution is treated with pancreatic ribonuclease to hydrolyze RNA and protease to degrade proteins. Residual proteins and oligopeptides are extracted with an organic solvent, such as phenol or an equal mixture of phenol and chloroform. Most of the protein will denature and enter the organic phase or precipitate at the interface of the organic and aqueous phases, this phase separation being accomplished by means of centrifugation. The clear, viscous aqueous phase containing the DNA is then removed. With the addition of alcohol, the DNA precipitates out of the aqueous phase as a white fibrous material and can be spooled onto a glass rod. Precipitation from alcohol serves to concentrate the high molecular weight DNA while removing the small oligonucleotides of DNA and RNA, detergent, and the organic solvent used in the removal of proteins. Residual detergent and salts can be removed by dialysis of the resuspended DNA solution against the suitable buffer. In some instances, it may be desirable to further purify the DNA by centrifugation on isopycnic cesium chloride gradients, or by hydroxylapatite chromatography. In the above process for chromosomal DNA, typical protocols often require at least two days for the DNA extraction and purificaiton process. (See Recombinant Techniques by Raymond L. Rodrigues, and Robert C. TAct, 1983, p.162).

During the purification of extrachromosomal elements of prokaryotic DNA, including plasmids and bacteriophage, it is desirable to minimize the amount of chromosomal DNA contaminating the preparation. With Bacteriophage, this is often accomplished by first purifying the phage particles from the infected bacteria, then treating the purified phage particles with protease and/or phenol to release the bacteriophage DNA. Further purification of the DNA is accomplished by means similar to those described for chromosomal DNA. Due to its size, however, precipitated bacteriophage and plasmid DNA cannot be spooled out on a glass rod and is therefore generally recovered by centrifugation. Again, three days is not atypical for the entire isolation arid purification process.

For eukaryotic cells, isolation arid purification of total cellular DNA is often achieved by a modification of the detergent lysis procedure described above for bacteria. The key difference is that typically cell lysis arid digestion of cellular proteins are accomplished using proteinase K in the presence of the detergent. (See M. Gross-Bellard, P. Oudet, and P. Chambon, Eur. J. Biochem., 36 (1973) 32-38; N. Blin, and D.W. Stafford, Nuc. Acid. Res., 3 (1976) 2303-2308; and D. J. Law, P.M. Frossard and D. L. Ruchnagel, Gene, 28 (1984) 153-158. The proteinase K is then removed by extraction of the lysate with phenol or a phenol/chloroform mixture. Typically, in the mixing process as for the extraction of bacterial DNA, the lysate/phenol or lysate/phenol-chloroform forms an emulsion, the aqueous and organic phases of which are separated by centrifugation. The upper, or aqueous, phase containing the DNA is then poured off or removed using a pipette, and this essentially protein-free lysate is dialyzed to remove small molecular weight cellular contaminants and residual phenol.

In the above approaches, a major limitation on the extraction which critically limits the ability to automate the process, is the need for centrifugation to separate the aqueous and organic phases during the phenol extraction. Often several extractions are required to achieve the desired purity, each one requiring centrifugation. Largely due to these various centrifugations, the work is performed manually and is therefore expensive. Also these configurations make automating of the extraction process difficult and expensive.

In accordance with the present invention, an automated apparatus is provided which implements a new method of extracting and purifying nucleic acids from cells without the use of centrifugation. The apparatus comprises an apparatus for extracting nucleic acids comprising:
reaction vessel means for holding a sample to be extracted, the reaction vessel means being of a flow through type comprising a tube having an opening at one end for loading said sample, hereinafter the top end, and an opening at the other end, hereinafter the bottom end;
rocker means for mounting and then rocking and rotating about a horizontal axis the reaction vessel means, fluid delivery means, waste manifold means and heater means;
fluid delivery means for controlled delivery of proteinase K, a lysis buffer and a phenol based solvent system to the reaction vessel means;
waste manifold means for draining fluids from the reaction vessel means through the other end; and
heater means for heating the reaction vessel means.

Furthermore, the method for extracting nucleic acids using the above apparatus comprises
rocking the reaction vessel means to a position wherein the top end of the reaction vessel for receiving the sample is lower than the other end;
venting the other end to the waste manifold means;
adding a lysis buffer and proteinase K;
rocking the reaction vessel means to a position wherein the end for top end receiving sample is higher than the other end;
adding sample to the reaction vessel means;
rocking the reaction vessel means to promote digestion of the sample to create a lysate;
rocking the reaction vessel means to a position wherein the other end is higher than the top end;
venting the other end to the waste manifold means;
adding a phenol based solvent system to the reaction vessel means;
mixing the lysate with the solvent to create an emulsion of extracted amino-acids and proteins;
heating the reaction vessel means to effect nucleic acid phase separation of the emulsion into aqueous nucleic acid containing phase and an organic phase;
educting the organic phase;
concentrating aqueous nucleic acid containing phase; and
collecting the concentrated nucleic acid portion.

The heating of the emulsion and optionally using a high salt concentration lysis buffer and increasing the surface area of the emulsion results in phase separation in 2 to 8 minutes and thus totally eliminates the need for centrifugation.

A computer system can be used for controlling the heating system, the rocker means, and the delivery system, for timing the flow of the reagents into the reaction vessel to control volume, for monitoring the temperature in the reaction vessel, and for monitoring the flow of the organic phase out of the reaction vessel. The computer also serves as the master timer, timing the mixing by the rocker means and the wait time during phase separation, and operates according to a preselected instruction set based on the above method.

The reaction vessel and dialysis apparatus are operated independently, but the dialysis system is configured to mate with the reaction vessel for easy transfer of material from one to the other. Also, the apparatus is especially adapted for normal ethanol precipitation of the nucleic acids in the reaction vessel.

A specific embodiment of the invention will now be described by way of example only with reference to the accompanying drawings in which:

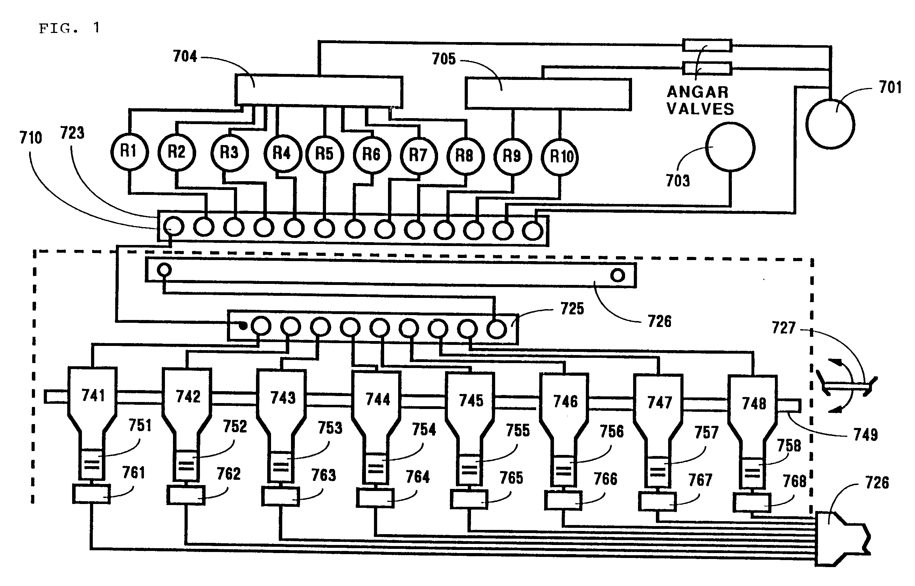

Fig. 1 shows a schematic representation of the apparatus of the invention.
Fig. 2 shows a cross-sectional view of a reaction vessel and how it is sealed.
Fig. 3 shows three different shapes of reaction vessel.
Fig. 4 shows a side view of a rocker apparatus.
Fig. 5A shows an expanded view of a dialysis cartridge.
Fig. 5B shows the completed cartridge of Fig. 5A.
Fig. 6 shows a method used to fill the dialysis cartridge of Fig. 5B.
Fig. 7 shows a front view of dialysis cartridges attached to the rocker apparatus.
Fig. 8 shows a precipitate collection unit.

### Definitions

For the purpose of the description of the invention, the following definitions will apply:

An "emulsion" is a mixture of two immiscible liquids which are kept in suspension, one within the other. In the context of the extraction of nucleic acids from cells, after cell lysis and mixing of the lysate with a phenol-based solvent system, an emulsion is formed, the consituent fluids of which are an aqueous phase containing the nucleic acids, and an organic phase containing the phenol, denatured proteins, and lipids and other cell constituents. In addition to nucleic acids, the aqueous phase also typically contains impurities which in many situations must be removed before further in vitro manipulations can be performed. These impurities include trace amounts of the phenol-based solvent system, many small molecular weight cellular constituents such as carbohydrates, amino acids, and smaller nucleic acids such as nucleosides and nucleotides (usually referred to as the cell sap).

"Dialysis" is a separation process that depends on the differential transport of solutes of different sizes across a porous barrier separating two liquids where the driving force is a concentration gradient only. In the extraction of nucleic acids, dialysis is often used to remove the impurities in the aqueous phase of the emulsion.

"Restriction" is the selective endonucleitic cleaving of DNA at unique base sequence sites. Generally, restrictability is considered a stringent test for DNA purity.

The apparatus is designed to mate with a dialysis cassette, hereinafter called a dialysette^{(TM)} cartridge, so that after the extraction the dialysis can be performed independently if desired, for example at a reduced temperature. It is also adapted for use with methods of normal ethanol precipitation of nucleic acids.

The apparatus includes a source of high pressure 701, typically about 10 psi guage, a source of low pressure 703, typically about 1.5 psi guage, a gas manifold 704 for the aqueous reagents, and a gas manifold 705 for the organic reagents. A typical selection of aqueous reagents is as follows: vessel R1 contains proteinase K; vessel R2 contains lysozyme; vessel R3 contains lysis buffer e. g. 4M urea, 0.2 NaCl, 100mM Tris-HCl pH 8.0, 0.5% n-lauroyolsarcosine, 10mM CDTA; vessel R4 contains 3M sodium acetate pH 5.5; vessel R5 is an extra vessel to be used as desired; vessel R6 contains 70% ethanol; and vessel R7 contains water. The organic reagents are as follows: vessel R8 contains ethanol; vessel R9 contains phenol/chloroform in a 50/50 ratio; and vessel R10 contains chloroform. An electrically operated valve block 723 is used to control the flow of reagents out of the various vessels R1-R10, with metering based on time and pressure as before. Port 710 in valve block 723 is connected to a valve block 725 which directs the flow of the various reagents to eight reaction vessels 741-748. A strip heater 749 is attached to each vessel to provide the desired temperature control during extraction. Valve block 725 and reaction vessels 741-748 are also coupled to a waste manifold 726. The reaction vessels 741-748, the waste manifold 726, strip heater 749, and the valve black 725 are all mounted on a rocker apparatus and are all rocked simultaneously about an axis 727 by a motor (not shown). The oscillation about axis 727 during the extraction process is quite slow, about one per second, typically through an angle of 50° to 60°. Also, the apparatus is rocked so that the reaction vessels are in a full horizontal position during phase separation.

The material in the reaction vessels can be removed by gravity flow since the vessels are of a flow through type, or it can be pressured out the bottom if so desired. Also at the bottom of reaction vessels 741-748 are conductivity cells 751-758, one for each vessel, for monitoring when the organic phase has been educted. Flow out of the reaction vessels is controlled by angar valves 761-768.

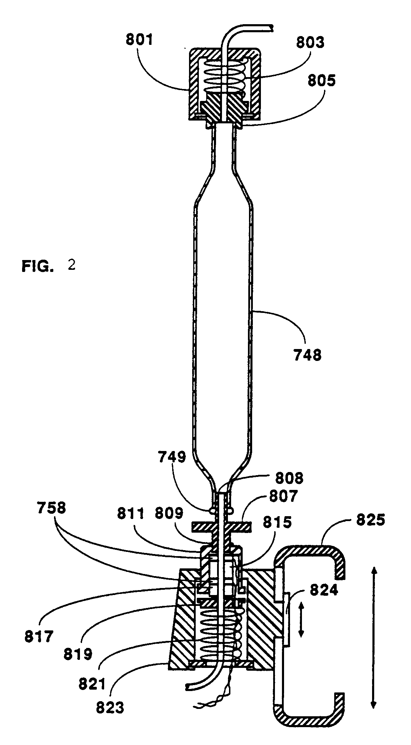

Fig. 2 shows a cutaway side view of a portion of the apparatus for holding the reaction vessels on the rocker apparatus. A cap 801 holds a springloaded seal 805 tightly against the top of reaction vessel 748. At the bottom, a run connector 807, typically polyethylene, seals the reaction vessel with a male Luer-type fitting 808. The run connector is removable to facilitate washing of the reaction vessel. In that instance, lip 749 of reaction vessel 748 rests against an O-ring 809, so that the entire interior of the reaction vessel can be cleaned and there is no dead space which can contribute to contamination.

A barrel 811 which moves inside a housing 823 holds run connector 807 in place. The conductivity cell 758 is typically gold coated for maximum sensitivity i.e. to provide corrosion resistance. The two electrodes of the cell are separated by a teflon spacer 815. Another teflon spacer 817 holds the cell in place against a holding ring 819 which is loaded by a spring 821 thereby loading the entire assembly against the reaction vessel 748. Also, housing 823 is slideably attached to a mounting 825 via a pin 824 which slides up and down in mounting 825 to effect a tight seal of the run connector to the reaction vessel.

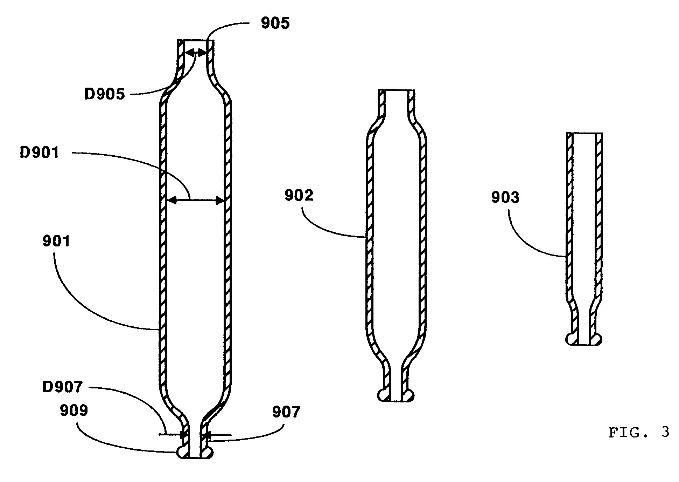

Shown in Fig. 3 are three examples of reaction vessels designed for use in this second embodiment. Vessel 901 is a 14 ml vessel which is constructed of glass about 6 inches long and has a maximum internal diameter D901 of about 0.6 inches, an aspect ratio of about 10:1. Top 905 of the vessel 901 is cut square to seat firmly against springloaded seal 805, without providing any dead space to collect residual DNA. Since materials to be digested are introduced through top 905, the internal diameter D905 of the top is relatively large, about 0.25 inches. At the bottom of the vessel 901, the vessel necks down to an internal diameter D907 of about 0.156 inches and is ground internally to an angle of 1.7^{o} to provide a female Leur-type fitting 907. At the very bottom of the vessel, a lip 909 is provided to effect a seal with O-ring 809 during cleaning. Reaction vessel 902 is a 7 ml vessel having an overall length of about 4.5 inches and a maximum internal diameter of 0.45 inches to provide an aspect ratio of about 10:1. Vessel 902 is provided with a female Leur-type fitting and a lip at the bottom as in vessel 901. Similarly the top is cut square and is the same size as that of vessel 901 to seat against seal 805. Reaction vessel 903 is a 3.5 ml vessel about 3.6 inches long and having a maximum internal diameter of about 0.36 inches, again an aspect ratio of about 10:1. The top and bottom of the vessel match those of vessels 901 and 902. The aspect ratio of 10:1 in each of these vessels is to provide a practical ratio for the cross-sectional area of a fluid in the vessels in the upright position to the area of a fluid in the vessel when it is in the horizontal position, thus, optimizing the step of phase separation. Even larger aspect ratios are desirable for phase separation, however, aspect ratios which are substantially larger than about 15:1 are not practical for other steps of the process, e. g. when mixing reagents in the vessel. Also, lower aspect ratios than 10:1 can be used, but lower than about 6.5:1 the phase separation is not significantly enhanced.

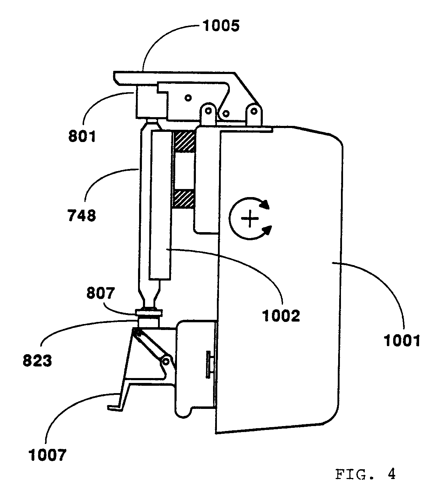

Fig. 4 shows a side view of the rocker apparatus 1001 in its normal upright position. The rocker apparatus carries a cradle 1002 to which the reaction vessels are attached, typically by thermal epoxy. The cradle also holds the strip heater 749 in contact with the vessels. Mount 825 is slideably attached to rocker apparatus 1001 and holds the pin 824 (not shown) arid housing 823 to permit the different length reaction vessels to be held in place. Cap 801 is held in place by a latch 1005 attached to rocker apparatus 1001 which clamps down on the cap and locks it into place to effect a tight seal between the springloaded seal 805 (not shown) arid the reaction vessel 748. Run conector 807 is held firmly in place against the reaction vessel by a lever actuator 1007 which moves housing 823 up and down one side of mount 825 as described earlier.

The method of the invention involves the rocker apparatus 1001 being inverted, inverting the reaction vessels 741-748, and the vessels are vented to waste manifold 726, one at a time to avoid cross-contamination from vessel to vessel, and similarly for all further operations except a purge cycle, to be discussed later. Lysis buffer and proteinase K are then added via valve blocks 723 and 725 to the desired number of reaction vessels. The vessels are then preincubated at about 60° to remove any residual nuclease activity which may be present in the proteinase K for 5-10 minutes, while gently rocking the vessels. (To simplify the rest of the discussion, the process will be described relative to only one reaction vessel, vessel 748.) A high salt concentration lysis buffer may be employed, the salt increasing the ionic strength. A preferred lysis buffer contains 4M urea; 0.5M NaCl; 0.5% SDS, 50mM Tris-HC1, and 10mM EDTA, pH 8.0. An 8M urea concentration corresponds approximately to saturation and so represents an upper limit. Lower urea concentrations can also be used, and 4M is preferred for best efficiency. A lower limit appears to be about 2M urea. Also, other chaotropic agents, e.g. guanadine hydrochloride, may be substituted for urea. The concentration of the detergent SDS can also be varied, typically from 0.5% to as much as 2%, but a concentration of about 0.5% appears to be more than adequate for most types of mammalian cells. Other detergents such as Triton X-100 (TM), Nonedit (TM), and lauroyolsarcosine may also be used. Similarly, the high salt concentration can be varied from 0.1M, which considerably slows down phase separation to as high as 2M, which does not seem to appreciably increase the rate of phase separation over that obtained with the preferred 0.5M NaC1 solution. Other salts, e.g., KC1, may also be used, the preferred concentration depending on the ionic strength of the salt used. Also, chelating agents other than EDTA may be used, for example 8-hydroxyquinoline, and in some instances, the chelating agent may be omitted. The Tris-HC1 serves as a buffer.

The rocker apparatus is then rocked back to its normal upright position and the cap 801 is removed by raising latch 1005. A nucleic acid sample is then added to the reaction vessel from the top and the latch 1005 is closed pressing cap 801 firmly onto the reaction vessel. The rocking apparatus is then moved to a nominal position 90° relative to its usual upright position, and the reaction vessel is rocked back and forth about the horizontal position while heating nominally to 60° to digest the sample. After digestion is complete (0.5 to 3 hrs) the rocker apparatus is turned upside down and the reaction vessel is vented to waste. The phenol/chloroform solvent is then delivered to the reaction vessel with the lysate via valve blocks 723 and 725 and the lysate and solvent are gently mixed by rocking back and forth about the horizontal position for about 15 minutes to extract the contaminating protein from the nucleic acids.

Preferably the phenol based solvent system contains phenol/chloroform/isoamyl alcohol at ratios of about 50:48:2, the phenol for denaturing and extracting the proteins, the chloroform to increase the hydrophobicity, and to ensure that the DNA remains in an aqueous phase and the isoamyl alcohol to serve primarily as an antisurfactant (antifoaming agent). Variations on this organic solvent system may be used, such as replacing the phenol/chloroform/isoamyl alcohol system with a phenol saturated with Tris-HC1 buffer, pH 8.0, or replacing the chloroform with methylene chloride, but the phenol/chloroform/isoamyl alcohol system is preferred because of its efficiency in effecting the desired phase separation and because the DNA does not get lost in the interface between the organic and aqueous phases, as can happen with the system using phenol saturated with buffer. Similarly, the precise volumetric ratios (i.e., 50:48:2) of this preferred phenol based solvent system can be varied, but too low of a concentration of chloroform often permits the DNA to enter the organic phase rather than remain in the aqueous phase. As a general rule, a ratio of phenol to chloroform of about 1:1 seems to provide optimum performance. Similarly, too low of a concentration of the antisurfactant can result in foaming which can clog the tubes.

Once the extraction is complete, the rocker apparatus is stopped and the reaction vessel is held in a horizontal position for about 5 minutes while the vessel is heated to bring about the phase separation.

The vessel is preferably heated to a temperature of between 35°C and 55°C, more preferably between 45°C and 55°C, and most preferably to about 55°C, that latter temperature being close to the boiling point of chloroform. Increasing the surface area is typically done by positioning the reaction vessel containing the emulsion on its side which increases the cross-sectional area of the interface between the lysate and organic phase and decreases the depth of the two phases. The combination of the high concentration of salt, the large interface area, and the process of heating during this step causes the emulsion to separate into a two-phase system in 2 to 8 minutes, thus totally eliminating the need for centrifugation.

Once phase separation has occurred, the rocker apparatus 1001 is rocked to a position so that the bottom of the reaction vessel is about 10° above the horizontal and the Angar valve 768 is vented to waste manifold 726 to relieve the pressure built up by heating. The Angar valve 768 is then closed and the vessel is moved to the upright position and the lower phenol phase is educted to waste manifold 726. The conductivity cell 758 is used to detect the change in phase in order to stop educting material from the reaction vessel. The above extraction process is then repeated as before until the upper phase is protein free. Typically only one additional extraction is required for the isolation of nucleic acids from lymphocytes, whereas for some other cell types, for example liver, as many as three additional extractions may be required. Typically, the final extraction is performed with chloroform alone which removes most of the residual phenol, rather than with the phenol/chloroform/isoamyl alcohol mixture. The time for these subsequent extraction typically remain about 10-15 minutes. However, the subsequent phase separation typically occurs in shorter times, 2-3 minutes.

Once the above extraction procedure is complete, either of two methods of concentrating the aqueous phase containing the nucleic acids is available, dialysis or ethanol precipitation.

Optionally, the aqueous phase can be treated with either RNase or DNase, and the extraction repeated to leave only DNA or RNA, respectively, in the aqueous phase.

The purified sample may then be collected.

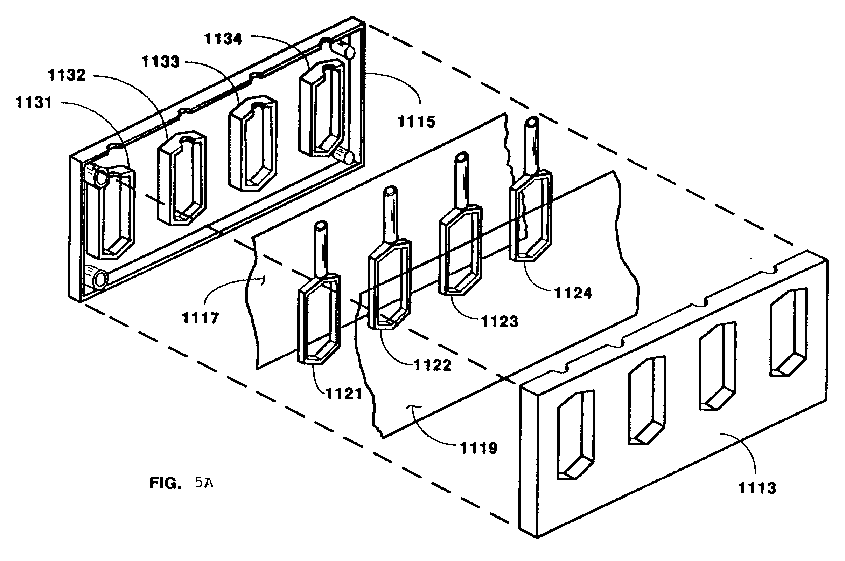

Shown in Fig. 5A is a construction drawing of a Dialysette (TM) cartridge 1100 especially designed to fit onto the female Luer-type fittings at the bottom of the reaction vessels. The cartridge is made up of two frame halves 1113 and 1115 between which are sandwiched two dialysis membranes 1117 and 1119 and a set of gaskets 1121, 1122, 1123 and 1124, the gaskets being sandwiched between the dialysis membranes. Each of the frame halves has a series of raised windows 1131-1134 which, when the frame halves are fastened together, clamp the dialysis membranes and gaskets firmly in place providing four reservoirs defined by the space between the membranes which is provided by gasket 1121-1124.

Fig. 5B shows a completed structure with the four reservoirs 1141-1144. To fill the reservoirs, the gaskets are provided with a fill tube which have male Luer-type fittings 1151-1154 at the top. Also, each of the fill tubes is spaced apart precisely the same distance that the reaction vessels are spaced apart in order to fit therein.

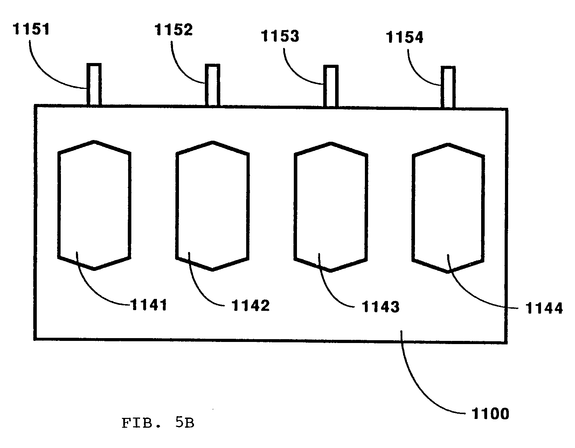

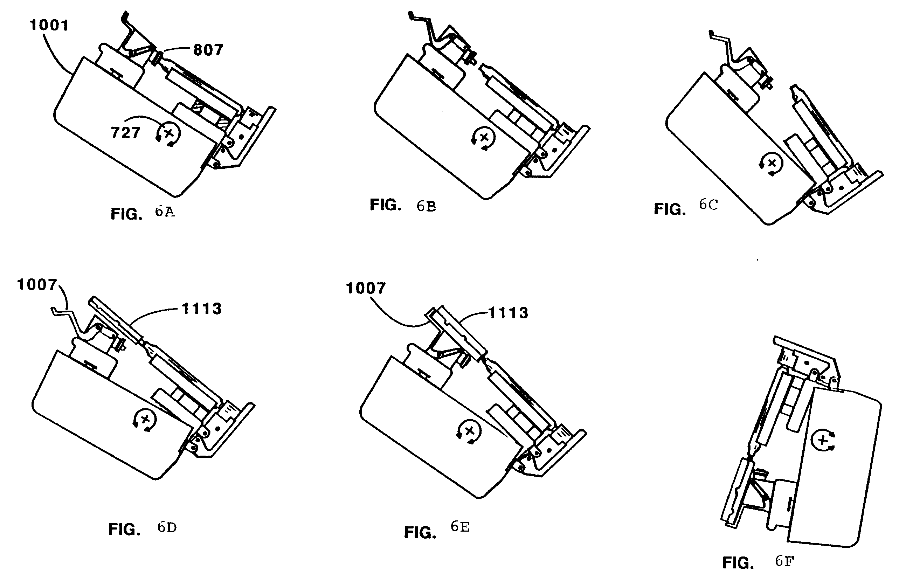

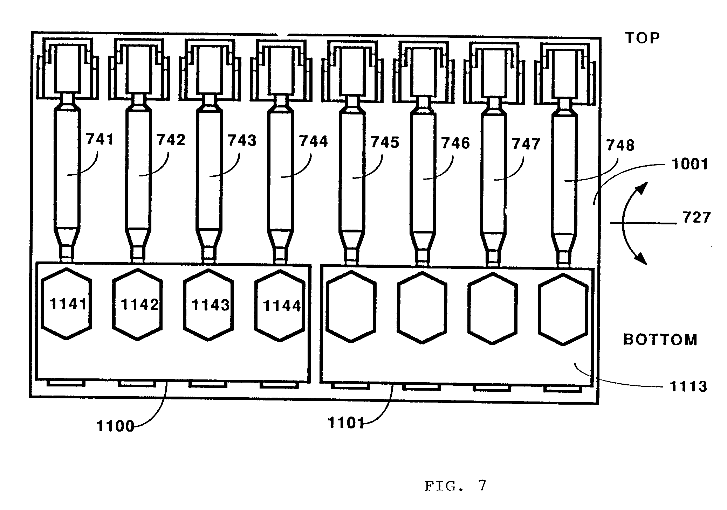

Figs. 6A-6B illustrate the method used to attach and fill the dialysette cartridges. First, the Dialysette cartridge rocked to a semi-upright position as shown in Fig. 6A. The reaction vessels are then opened, as illustrated in Fig. 6B, and the vessels are swung away from the rocker apparatus 1001 somewhat, as illustrated in Fig. 6C, in order to attach the cartridge. The Dialysette cartridge 1113 is then attached as in Fig. 6D, and clamped into place as in Fig. 6E with lever actuator 1007, and the other corresponding lever actuators which are used for the other reaction vessels. The rocker apparatus is then rocked back to the position illustrated in Fig. 6F, thereby filling the reservoirs in the Dialysette cartridge.

Fig. 7 shows a front view of the rocker apparatus with dialysette cartridges 1100 and 1101 attached to the reaction vessels 741-748. Once the Dialysette cartridges are filled, they are removed and plugged and standard dialysis procedures are used to concentrate and purify the extracted nucleic acids. For a detailed description of the Dialysette cartridge see copending application entitled "Dialysette (TM) Dialysis Cartridge" filed April 10, 1986 by G. Richard Cathcart, et al.

The other approach to concentrate the nucleic acids, i.e. ethanol precipitation, is also enhanced by the structure of the apparatus. To concentrate the nucleic acids in vessel 748 by standard ethanol precipitation, a volume of sodium acetate is added to the reaction vessel from reagent vessel R4 which is equal to 0.1 the volume of the remaining lysate. Then two volumes of punctilious ethanol from reagent vessel R8 is added and the vessel is gently rocked to precipitate nucleic acids. The two volumes of ethanol correspond to twice the volume of the lysate and sodium acetate together.

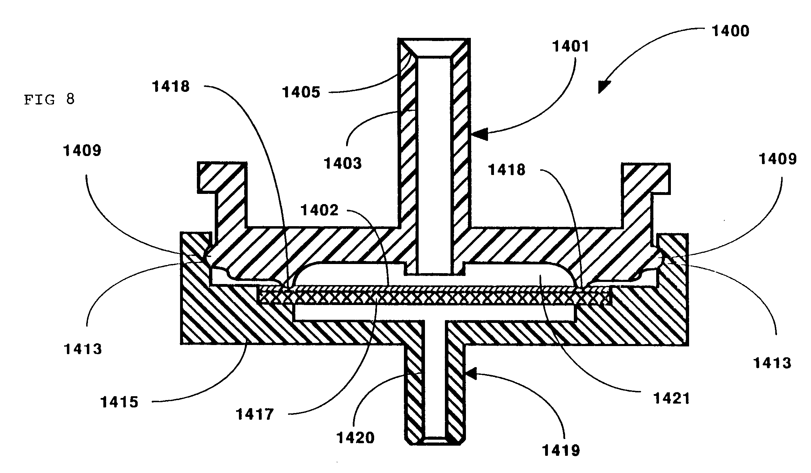

To collect the precipitated nucleic acids, a special collection device known hereinafter as a Precipitette ^{(TM)} collection unit 1400 is used. Fig. 8 shows a cross-section of the collection unit 1400. The unit is made up of a circular top portion 1401 which has an upward extending element that at the very top has the form of a male Luer-type fitting to fit into the bottom of a reaction vessel. A circular tube 1403 runs through top portion 1401 arid has a smooth chamfered surface 1405 in order to inhibit the collection of nucleic acids on the surface of the unit. The top portion 1401 has a snapping ring 1409 which snaps into circular detent 1413 located in a bottom portion 1415 in order to hold the top portion 1401 and bottom portion 1415 together. Sandwiched between the top and bottom portions is a support mesh 1417 for holding a filter 1402 thereon. A circular protrusion 1418 presses the filter 1402 against the mesh 1417 and holds both filter and mesh in place in the unit. A bottom portion 1419 is provided which fits directly into barrel 811 where the run connector 807 is normally attached. Also, bottom portion 1419 is provided with a tube 1420 in order to connect the unit to waste. A volume 1421 between the bottom of top portion 1401 and the mesh is provided to collect the precipitated nucleic acids.

To use the Precipitette ^{(TM)} collection unit 1400, once the ethanol precipitation has been, performed, the rocker apparatus is rocked to its upside down position. The run connectors are removed and the Precipitette collection units are installed in their places. The rocker unit is then vigorously rocked to its normal upright position while at the same time during this down stroke pulsing in air and ethanol to flush the precipitates from the sides of the reaction vessels and into the Precipitette collection units. The pulsing of the air and ethanol is performed in alternate cycles, the air typically for about 600 msec. at a time and the ethanol for about 300 msec. at a time although these times can vary depending on the size of the reaction vessel being cleared. As the precipitate is flushed from the reaction vessels, it is collected in filters 1402 in the collection units. If desired, the precipitates can be further washed by filling the reaction vessels with ethanol and draining them to waste through the collection units. The precipitate can then be collected by removing the filters from the collection units.

Following either dialysis or precipitation, the reaction vessels must be scrupulously cleaned before they can be reused, since a piece of left over DNA could contaminate a subsequent extractate and could get replicated over and over in future cloning experiments. To accomplish this cleaning, a purge cycle is instituted where each vessel is purged independently to avoid cross-contamination. This sequence is as follows. First, each vessel is rinsed by adding water which is heated in the vessel to about 60^{o}C. Each vessel is rocked vigorously and the hot water is blown to waste, one at a time. Then 6 normal nitric acid is added to each vessel and heated to about 60^{o}C while rocking vigorously. Then the nitric acid is blown to waste one vessel at a time. Each vessel is then rinsed with water, heated to 60^{o}C, rocked vigorously, and then the water is blown to waste again one at a time, A 50/50 mixture of Lysis buffer and water is added to each vessel to neutralize the nitric acid, heated to 60^{o}C, and the vessels rocked and blown to waste as before. Then each vessel is rinsed twice with water just as in the first water rinse. Each of the above cycles requires approximately three minutes. Following the purge operation, the reaction vessels are again ready for use.

At the most basic level, software control of the extraction apparatus is a matter of opening and closing valves and turning switches on and off at the proper times to achieve the desired flows of the various materials from one vessel to another and to perform the required operations. The fact that the method of the invention is a sequence of steps lends itself conveniently to software control.

### Examples. Preparation of DNA from Human Lymphocytes

Lymphocytes are first washed from one unit of whole blood and are resuspended in 4 ml balanced salt solution. (Balanced salt solution is made up of 1 volume of solution A and 9 volumes of a solution B, where solution A is 0.1% glucose, 5x10⁻¹⁰M CaCl₂, 9.8x10⁻⁴M MgCl₂, 5.4x10⁻³ , KCl, 0.145M Tris-HCl, pH 7.6; and Solution B is 0.14M NaCl.) Then 0.35 ml of the lymphocyte suspension above is mixed with 4 ml lysis buffer (1M NaCl, 1% SDS, 8M urea, 10 mM EDTA, 50 mM Tris-HCl, pH 8.0), and 1 mg of proteinase K in 0.65 ml lysis buffer to obtain a total volume of 5 ml. The digestion is performed in a conical tube (extraction vessel 11) as described earlier, at 55° C for 3 hours. About 5 ml of phenol/chloroform/isoamyl alcohol 50:48:2 is added to the tube and the two phases are mixed according to the protocol for 20 minutes. The extraction vessel is then rotated to the horizontal position for 10 minutes at 55° C to allow the phases to separate. The extraction vessel is then rotated slowly back up to the vertical position over a period of about 10 seconds, and the lower organic layer is removed to waste. A second extraction is performed with the phenol/chloroform/isoamyl alcohol mixture and a third extraction is performed using 5 ml of chloroform. The chloroform is removed to waste and the aqueous DNA-containing layer is pressure transferred to a dialysis bag, such as dialysis bag 311. This aqueous solution is then pressure dialyzed according to the protocol until A270 is below 0.01. The final DNA solution is then educted from the bag and collected.

Following this process yields about 1 ml of DNA solution containing about 250 micrograms of DNA. The resulting solution has an absorbance ratio A230:A260 of 0.52 and an absorbance ratio A260:A280 of 1.90, demonstrating very high purity. (For absolutely pure DNA, the absorbance ratio A230:A260 is 0.5±0.05 and A260:A280 is 1.9±0.1.) Analysis of the sample using a 0.8% agarose gel and standard ethidium using a staining techniques shows a single band with a size greater then 50kbase pairs (i.e., greater than 3.5x10⁷ daltons). Also most importantly, digestion with the enzyme, Eco RI is positive, indicating that the DNA is restrictable and therefore restrictability being therefor a very stringent test for DNA purity.

Variations on the above example demonstrate the importance of heating and increasing the surface area to effect the phase separation step. For example, for human lymphocytes, if the extraction vessel is not heated, but is maintained at room temperature, and the extraction vessel is not rotated to a horizontal position, phase separation typically requires over 60 minutes. If instead the extraction vessel is heated to 55° C but is not also rotated to the horizontal position, the phase separation requires over 4 minutes. With heating to 55° C and rotation of the extraction vessel to the horizontal position, the phase separation typically requires only about 2.5 minutes. In each of these variations, however, the high salt content is enhancing the rate of phase separation by approximately a factor of two.

While there has been shown and described a preferred embodiment of the apparatus and method of the present invention, it will be apparent to those skilled in the art that many charges and modifications may be made without departing from the invention in its broader aspects. For example, it is apparent that the extraction vessel need not be rotated to a horizontal position to speed up phase separation, although it does have a major influence. Similarly, the phase separation can be performed at a temperature below the preferred range of 45° C to 55° C, but it will proceed at a slower rate, and the further below that range the slower the rate. In terms of apparatus it will be apparent that automated devices according to the invention can be constructed with either more or fewer extraction vessels, reagent vessels, and dialysis bags. Also, some valves may be conveniently placed at different locations in the apparatus, for example valve block 52 may be placed ahead of conductivity meter 55. However, this would result in some loss of the aqueous phase when the flow is stopped. In addition, the specific model numbers chosen for the various pieces of apparatus included in, the automated extraction system are not meant to be restrictive as to the particular models which can be used, but are offered by way of example only.

## Claims

1. An apparatus for extracting nucleic acids comprising:
reaction vessel means (741 to 748) for holding a sample to be extracted, the reaction vessel means being of a flow through type comprising a tube having an opening at one end for loading said sample, hereinafter the top end, and an opening at the other end, hereinafter the bottom end;
rocker means (1001) for mounting and then rocking and rotating about a horizontal axis the reaction vessel means (741 to 748), fluid delivery means (723,725), waste manifold means (726) and heater means (749);
fluid delivery means (723,725) for controlled delivery of proteinase K, a lysis buffer and a phenol based solvent system to the reaction vessel means;
waste manifold means (726) for draining fluids from the reaction vessel means through the other end; and
heater means (749) for heating the reaction vessel means.

2. Apparatus according to claim 1, wherein the other end of the reaction vessel means (741 to 748) comprises a Liner-type fitting.

3. Apparatus according to claim 2, further comprising a dialysis cartridge means (1100,1101,1113) having a Luer-type fitting for connecting to and mating with the Luer-type fitting of the reaction vessel means (741 to 748) and for receiving extracted nucleic acid from the reaction vessel means for dialysis.

4. Apparatus according to claim 2 or claim 3, further comprising precipitate collection means (1400) for collecting nucleic acids precipitated in the reaction vessel means (741 to 748) the precipitate collection means (1400) having a Luer-type fitting for mating with the Luer-type fitting of the reaction vessel means (741 to 748).

5. Apparatus according to any preceding claim, further comprising a computer system for controlling the heater means, rocker means, fluid delivery means and waste manifold means, for controlling the flow of reagents into the reaction vessel means, for monitoring the temperature in the reaction vessel means and for monitoring the flow of fluids from the reaction vessel means.

6. A method of extracting nucleic acids from a sample using the apparatus according to claim 1, the method comprising
rocking the reaction vessel means to a position wherein the top end of the reaction vessel for receiving the sample is lower than the other end;
venting the other end to the waste manifold means;
adding a lysis buffer and proteinase K;
rocking the reaction vessel means to a position wherein the end for top end receiving sample is higher than the other end;
adding sample to the reaction vessel means;
rocking the reaction vessel means to promote digestion of the sample to create a lysate;
rocking the reaction vessel means to a position wherein the other end is higher than the top end;
venting the other end to the waste manifold means;
adding a phenol based solvent system to the reaction vessel means;
mixing the lysate with the solvent to create an emulsion of extracted amino-acids and proteins;
heating the reaction vessel means to effect nucleic acid phase separation of the emulsion into aqueous nucleic acid containing phase and an organic phase;
educting the organic phase;
concentrating the aqueous nucleic acid containing phase; and
collecting the concentrated nucleic acid portion.

## Patentansprüche

1. Vorrichtung zum Extrahieren von Nukleinsäuren, aufweisend:
eine Reaktionsgefäßeinrichtung (741 - 748) zum Aufnehmen einer zu extrahierenden Probe, wobei es sich bei dem Reaktionsgefäß um eine Durchflußeinrichtung handelt, welche ein Rohr aufweist mit einer Öffnung an dem einen Ende zur Aufnahme der Probe, nachfolgend als oberes Ende bezeichnet, und einer Öffnung an dem anderen Ende, nachfolgend als unteres Ende bezeichnet;
eine Schwenkeinrichtung (1001), um die Reaktionsgefäßeinrichtung (741 - 748), die Flüssigkeitsabgabeeinrichtung (723, 725), die Abfallsammelleitung (726) und die Heizeinrichtung (749) zu befestigen und zu verschwenken und um eine horizonte Achse zu drehen;
eine Flüssigkeitsabgabeeinrichtung (723, 725) zur gesteuerten Abgabe von Proteinase K, einem Lysis-Puffer und einem auf Phenol basierenden Lösemittelsystem für die Reaktionsgefäßeinrichtung;
eine Abfallsammelleitung (726) zum Ablassen von Flüssigkeiten oder Fluiden aus der Reaktionsgefäßeinrichtung durch das andere Ende; und
eine Heizeinrichtung (749) zur Erhitzung der Reaktionsgefäßeinrichtung.

2. Vorrichtung nach Anspruch 1, bei welcher das andere Ende der Reaktionsgefäßeinrichtung (741 - 748) eine Luer-Typ-Armatur aufweist.

3. Vorrichtung nach Anspruch 2, weiterhin aufweisend eine Dialysekartuscheneinrichtung (1100, 1101, 1113) mit einer Luer-Typ-Armatur zur Verbindung mit den und Anpassung an die Luer-Typ-Armaturen der Reaktionsgefäßeinrichtung (741 - 748) und zum Aufnehmen extrahierter Nukleinsäure aus der Reaktionsgefäßeinrichtung zur Dialyse.

4. Vorrichtung nach Anspruch 2 oder 3, weiterhin aufweisend eine Niederschlagsammeleinrichtung (1400) zum Aufsammeln der in der Reaktionsgefäßeinrichtung (741 - 748) abgesetzten Nukleinsäuren, wobei die Niederschlagsammeleinrichtung (1400) eine Luer-Typ-Armatur zur Anpassung an die Luer-Typ-Armatur der Reaktionsgefäßeinrichtung (741 - 748) aufweist.

5. Vorrichtung nach irgendeinem der vorangehenden Ansprüche, weiterhin aufweisend ein Computersystem zur Steuerung der Heizeinrichtung, der Schwenkeinrichtung, der Flüssigkeitsabgabeeinrichtung und der Abfallsammelleitung zur Steuerung des Flusses der Reagentien in die Reaktionsgefäßeinrichtung hinein, zur Überwachung der Temperatur in der Reaktionsgefäßeinrichtung und zum Festhalten der Strömung der Flüssigkeiten aus der Reaktionsgefäßeinrichtung heraus.

6. Verfahren zum Extrahieren von Nukleinsäuren aus einer Probe unter Verwendung der Vorrichtung nach Anspruch 1, wobei das Verfahren aufweist;
Verschwenken der Reaktionsgefäßeinrichtung in eine Position, in welcher das obere Ende des Reaktionsgefäßes zur Aufnahme der Probe tiefer als das andere Ende liegt;
Belüften des anderen Endes zur Abfallsammelleitung hin;
Hinzufügen eines Lysis-Puffers und Proteinase K;
Verschwenken der Reaktionsgefäßeinrichtung in eine Position, in welcher das obere Ende der zur Aufnahme der Probe höher als das andere Ende liegt;
Hinzufügen der Probe zur Reaktionsgefäßeinrichtung;
Schwingenlassen der Reaktionsgefäßeinrichtung, um die Verarbeitung der Probe zu fördern, um ein Lysat zu bilden;
Verschwenken der Reaktionsgefäßeinrichtung in eine Position, in welcher das andere Ende höher als das obere Ende liegt;
Belüften des anderen Endes zur Abfallsammeleinrichtung hin;
Hinzufügen eines auf Phenol basierenden Lösemittelsystems zu der Reaktionsgefäßeinrichtung;
Mischen des Lysats mit dem Lösungsmittel, um eine Emulsion extrahierter Aminosäuren und Proteine zu erzeugen;
Erhitzen der Reaktionsgefäßeinrichtung, um die Separation der Nukleinsäurephase der Emulsion in eine wässrige Nukleinsäure enthaltende Phase und eine organische Phase herbeizuführen;
Abziehen der organischen Phase;
Konzentrieren der die wässrige Nukleinsäure enthaltenden Phase; und
Auffangen des konzentrierten Nukleinsäureanteils.

## Revendications

1. Appareil pour extraire des acides nucléiques, comprenant:
des réacteurs (741 à 748) destinés à contenir un échantillon à extraire, les réacteurs étant du type à passage direct et comprenant un tube comportant une ouverture à une extrémité, destinée à introduire l'échantillon, et appelée ci-après l'extrémité supérieure, et une ouverture à l'autre extrémité, appelée ci-après l'extrémité inférieure;
un moyen d'oscillation (1001) pour monter puis faire osciller et tourner autour d'un axe horizontal les réacteurs (741 à 748), les moyens d'amenée de fluide (723, 725), le moyen collecteur de déchets (726) et l'élément chauffant (749);
un moyen d'amenée de fluide (723, 725) pour assurer un apport régulé de protéinaseK, d'un tampon de lyse et d'un système de solvant à base de phénol aux réacteurs;
un moyen collecteur de déchets (726) pour évacuer des fluides provenant du réacteur, par l'autre extrémité; et
un élément chauffant (749) destiné à chauffer les réacteurs.

2. Appareil selon la revendication1, dans lequel l'autre extrémité des réacteurs (741 à 748) comprend un raccord de type Luer.

3. Appareil selon la revendication2, comprenant en outre une cartouche de dialyse (1100, 1101, 1113) comportant un raccord de type Luer, pour raccordement au raccord de type Luer des réacteurs (741 à 748), et adaptation à ce dernier, et pour recevoir l'acide nucléique extrait, provenant du réacteur, pour le dialyser.

4. Appareil selon la revendication 2 ou 3, comprenant en outre un moyen collecteur de précipité (1400) destiné à recueillir des acides nucléiques précipités dans les réacteurs (741 à 748), le moyen collecteur de précipité (1400) possédant un raccord de type Luer, destiné à s'adapter au raccord de type Luer des réacteurs (741 à 748).

5. Appareil selon l'une quelconque des revendications précédentes, qui comprend en outre un système informatisé pour commander l'élément chauffant, le moyen d'oscillation, le moyen d'amenée de fluide et le moyen collecteur de déchets, pour réguler le débit des réactifs à l'intérieur des réacteurs, pour surveiller la température dans les réacteurs et pour surveiller le débit des fluides sortant des réacteurs.

6. Procédé pour extraire des acides nucléiques d'un échantillon, par utilisation de l'appareil selon la revendication1, procédé consistant
à faire osciller le réacteur jusqu'à une position dans laquelle l'extrémité supérieure du réacteur, destinée à recevoir l'échantillon, se trouve plus bas que l'autre extrémité;
à raccorder l'autre extrémité au moyen collecteur de déchets;
à ajouter un tampon de lyse et de la protéinaseK;
à basculer le réacteur jusqu'à une position dans laquelle l'extrémité supérieure, destinée à recevoir l'échantillon, est située plus haut que l'autre extrémité;
à introduire un échantillon dans le réacteur;
à faire basculer le réacteur pour favoriser la digestion de l'échantillon et créer un lysat;
à basculer le réacteur jusqu'à une position dans laquelle l'autre extrémité est située plus haut que l'extrémité supérieure;
à raccorder l'autre extrémité au moyen collecteur de déchets;
à introduire dans le réacteur un système solvant à base de phénol;
à mélanger le lysat avec solvant pour réaliser une émulsion d'acides aminés extraits et de protéines;
à chauffer le réacteur pour effectuer une séparation de la phase d'acides nucléiques de l'émulsion en une phase aqueuse contenant les acides nucléiques et en une phase organique;
à évacuer la phase organique;
à concentrer la phase aqueuse contenant les acides nucléiques; et
à recueillir la portion concentrée d'acides nucléiques.
